Europäisches Patentamt

European Patent Office

Office européen des brevets

(11) Publication number: **0 405 693 A1**

# EUROPEAN PATENT APPLICATION

(21) Application number: 90201725.0

(22) Date of filing: 28.06.90

(51) Int. Cl.5: **G01N 33/22, G01N 31/12**

(30) Priority: 30.06.89 NL 8901660

(43) Date of publication of application:
02.01.91 Bulletin 91/01

(84) Designated Contracting States:
AT BE CH DE DK ES FR GB GR IT LI NL SE

(71) Applicant: N.V. NEDERLANDSE GASUNIE
Postbus 19
NL-9700 MA Groningen(NL)

(72) Inventor: Dijkstra, Kees
Troelstralaan 63 D
NL-9722 JE Groningen(NL)

(74) Representative: Bleukx, Lucas Lodewijk Maria,
Ir. et al
OCTROOIBUREAU DSM Postbus 9
NL-6160 MA Geleen(NL)

(54) Method and device for determination of the wobbe index.

(57) The invention relates to a method for determination of the Wobbe index of a gas mixture, the gas mixture being supplied at a first flow rate through at least one flow opening from a supply section to a mixing chamber, an oxidation gas being supplied at a second flow rate through at least one flow opening from a supply section to the mixing chamber, in the mixing chamber a second gas mixture being formed and this second gas mixture being burnt in a combustion chamber, after which the air factor of the resulting combustion gas is measured, the pressure of the first gas mixture and that of the oxidation gas just before the mixing chamber being kept in a certain ratio by means of a pressure differential measurement controlling the flow rate of the first gas mixture and/or the oxidation gas, and in that the Wobbe index is determined on the basis of the air factor

Fig. 1

## METHOD AND DEVICE FOR DETERMINATION OF THE WOBBE INDEX

The invention relates to a method for determination of the Wobbe index of a gas mixture, the gas mixture being supplied at a first flow rate via at least one flow opening from a supply section to a mixing chamber, a second gas mixture being formed in the mixing chamber and this second gas mixture being burned in a combustion chamber, after which the air factor of the resulting combustion gas is measured.

Such a method is known, being employed in the so-called "comburimeter", as commercially marketed by Gaz de France.

In this known measurement device gas and air are supplied separately, via pressure and flow controllers, to a mixing chamber, resulting in a certain mixing ratio in this chamber.

The drawback of this method is that absolute control equipment is used, so that any deviations between the equipment used in the gas supply system and that for the air supply system give rise to standard errors in the Wobbe index measured. This phenomenon may also occur when such deviations arise as a consequence of ageing phenomena.

It is the object of the invention to provide a method of the above type in which these drawbacks are avoided.

According to the invention this object is achieved in that the pressure of the first gas mixture and that of the oxidation gas just before the mixing chamber are kept in a certain ratio by means of a pressure differential measurement that controls the flow rate of the first gas mixture and/or that of the oxidation gas, and in that the Wobbe index is determined on the basis of the air factor measured.

Other characteristics and advantages of the invention will become evident from the following description, in which reference is made to the attached drawings, where:

Figure 1 schematically represents a first device for carrying out the method according to the invention,

Figure 2 schematically represents a device for carrying out the method according to the invention in an embodiment that is changed relative to Figure 1,

Figure 3 is a perspective representation showing how the supply line and the mixing chamber can be separated in the device according to Figure 1,

Figure 4 is a partial section through the separation according to Figure 3,

Figure 5 is a perspective representation of a gas and air supply system as used in practice,

Figure 6 is a diagrammatical section through a pressure gauge applied in a device according to the invention, and

Figure 7 is an embodiment of a pressure gauge that is changed relative to Figure 6.

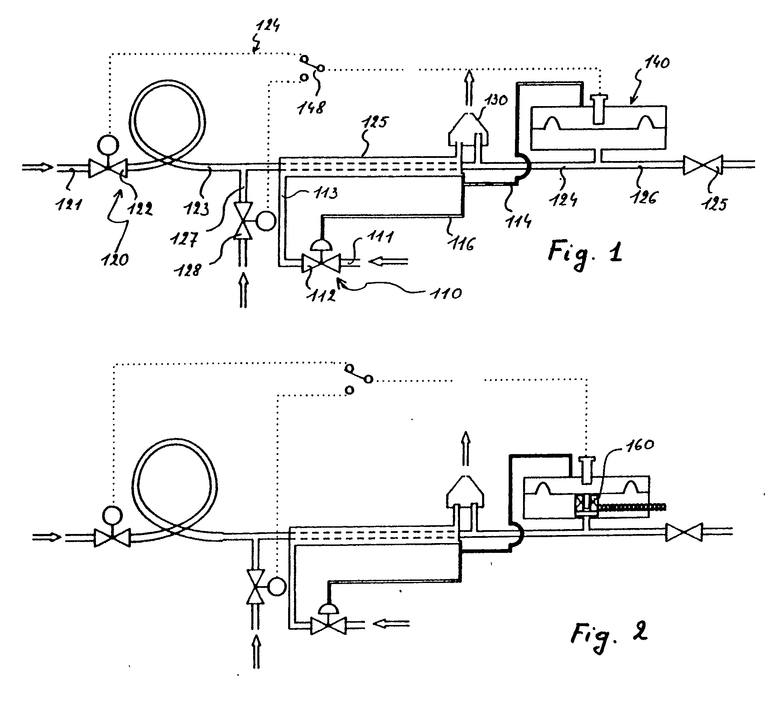

The device as shown in Figure 1 comprises a supply section 110 for the oxidation gas and a supply section 120 for the gas mixture of which the Wobbe index is to be determined. The oxidation gas may be air, but use may also be made of any other gas by which the gas mixture can be oxidized, such as pure oxygen, oxygen-enriched air, peroxides, etc.

The gas mixture may be natural gas, but in principle any other gas or gas mixture suitable for combustion purposes can be examined by means of the method according to the invention.

Oxidation gas supply section 110 comprises a supply line 111, which on one side is connected with an oxidation gas source supplying an oxidation gas at substantially constant pressure. On the other side supply line 111 is connected with a pressure control valve 112, via which the oxidation gas can flow into a pipe section 113. Pressure control valve 112 is used to control the gas pressure in pipe section 113.

Gas mixture supply section 120 comprises a supply line 121, which on one side is connected with a source of the gas mixture to be measured, and on the other with a pressure control valve 122, through which the gas mixture can flow to a pipe section 123. The setting of pressure control valve 122 is determined by means of a control circuit 124, the operation of which will below be described in further detail.

Pipe sections 113 and 123 are at least in part parallel and in heat-exchanging contact, for instance via a coaxial section 125. Around section 125 an electrical heating device may be provided, which can be used to heat the gas mixture and the oxidation gas to the same temperature. Pipe section 113 ends in a mixing chamber 130, as does pipe section 123. Mixing chamber 130 is further connected with a combustion chamber, not shown, which in its turn is connected with a measurement chamber, not shown, where a probe is installed, as described in NL-A-8802336. The probe determines the oxygen content in the measurement chamber. This probe may be of the type described in publication No. 730575 of Research Laboratories +. General Motors Corp., Sensor for On-Vehicle Detection of Engine Exhaust Gas Composition, by William J. Fleming, David S. Howarth and David S. Eddy. The signal generated by the probe is a direct measure of the amount of oxygen in the measurement chamber, which in its turn is a measure of the Wobbe index of the burnt gas.

The outlets of pipe sections 113 and 123 into mixing chamber 130 have been chosen such that the gas mixture formed in the mixing chamber always is richer in oxygen than needed for stoichiometric combustion.

Via a line 124 pipe section 123 is connected with one side of a differential pressure gauge 140, the other side of which is connected with pipe section 113 via a line 114. Line 124, in its turn, is connected with the atmosphere via a line 126, optionally a throttle valve being installed in between them, which may take the form of a capillary tube.

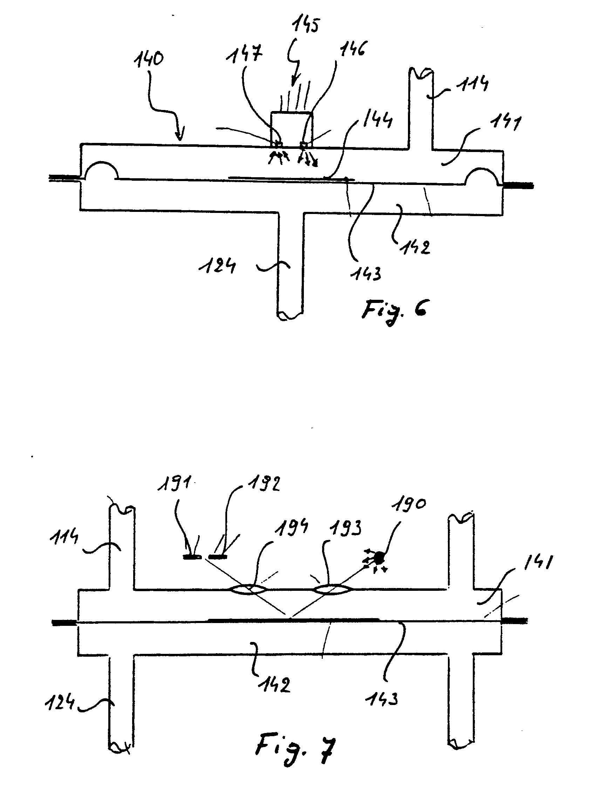

Differential pressure gauge 140 may be of the type as shown in more detail in Fig. 6, but it is evident that use can be made of any device suitable for measuring a pressure differential and converting this into a proportional signal.

As shown in Fig. 6, pressure gauge 140 consists of two chambers, 141 and 146, separated by a diaphragm 143, chambers 141 and 142 being connected to lines 114 and 124, respectively. On diaphragm 143 a reflecting plane 144 has been formed, which is opposite a photoelectric detection device 145 in the wall of chamber 141. This photoelectric detection device 1445 comprises a light source 146, for instance in the form of a LED, and a photo-sensitive element 147, for instance a photo-transistor, which are placed side by side such that at least part of the amount of light transmitted by a light source 17 reaches photo-transistor 147 after reflection by plane 144.

The output signal of photo-transistor 147 is compared with the set point and the resulting signal is used for setting valve 122.

Pressure gauge 140 operates in the following way. When the pressure in chamber 141 is the same as that in chamber 142, diaphragm 143 is in a central position, with a certain amount of light falling on photo-transistor 147 via plane 144. This generates an output signal, which corresponds with the set point. If the pressure in chamber 142 is higher than that in chamber 141, diaphragm 143 moves in the direction of the detection device 145. As a result, the amount of light falling onto photo-transistor 147 increases, resulting in a higher output signal, upon which, after comparison with the set point, a "positive" control signal is sent to valve 122. If the pressure in chamber 141 is higher than that in chamber 142, diaphragm 144 moves in the other direction, so that less light falls onto photo-transistor 147 and its output signal is thus reduced. After comparison with the set point this results in a "negative" signal for valve 122.

By preference pipe section 123 is a flexible hose of sufficient length, so that the measurement device need not always be placed in the immediate vicinity of the gas branch point.

Since valve 122 is a reducing valve and is of a capillary nature, the gas volume in line 123 is so small that this does not cause any appreciable delays in the measurement system.

Line 123 is further connected with a second supply line, 127, with reducing valve 128. Via this system a calibration gas can be supplied to mixing chamber 130. The electrical circuit by which the pressure measurement signal is passed to valve 122 incorporates a two-way switch 148 by which this signal can be fed back to valve 128.

In order to keep the pressure of the air supplied to mixing chamber 130 as constant as possible, a line 116 is provided, which on one side is connected with line 113 just before the latter ends in mixing chamber 130, and on the other with valve 112, so that valve 112 is controlled such that the air pressure in line 113 just before mixing chamber 130 is constant irrespective of the resistance in line 113.

The device described operates as follows. Gas and air are supplied through lines 120 and 110, respectively. Via pressure gauge 140 and control circuit 124 the pressures and in the coaxial section the temperatures of the air and of the gas are equalized. As a result, in the mixing chamber a gas-air mixture is formed at a certain mixing ratio (with an excess of air), which is subsequently burnt, following which the Wobbe index can be determined by measuring the oxygen excess. To this end, the probe can be calibrated using a gas of a known Wobbe index via valve 127 and after switching of switch 150.

In the embodiment as described with reference to Fig. 1 it is assumed that the probe signal is a direct indication of the Wobbe index. It is known that the sensitivity characteristics of the probe are such as to generate a maximum signal when the burnt gas mixture exhibits virtually stoichiometric ratios. In addition, the sensitivity characteristics may change due to ageing and temperature. For these reasons it may be desirable not to use the signal from the probe directly as an indication of the Wobbe index, but instead to control the supply of gases, air or natural gas such that always a stoichiometric mixture is supplied to the combustion chamber,

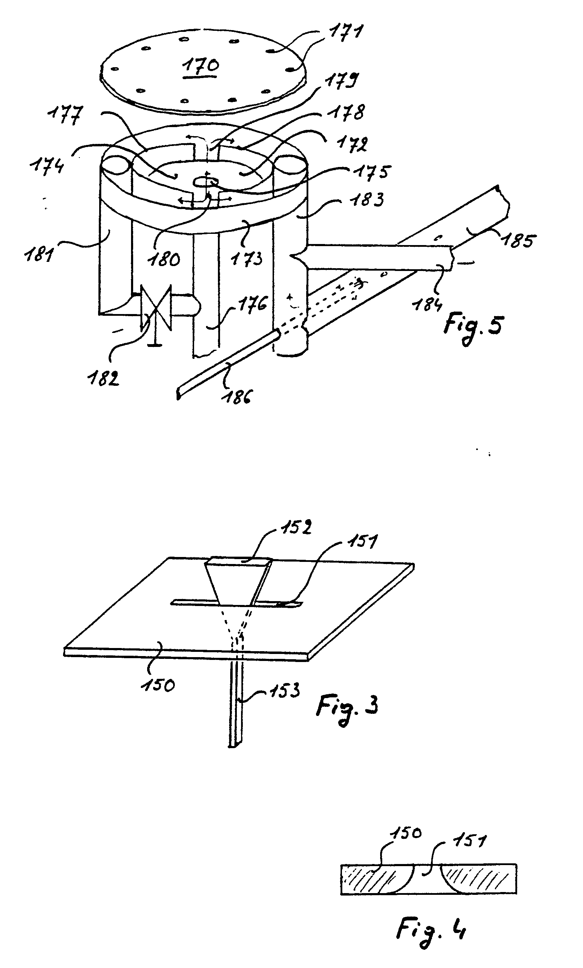

To this end the embodiment according to Fig. 1 can be modified in that the opening connecting line 125 and mixing chamber 130 is designed as shown in Figs. 3 and 4. A plate 150 forms the seal between line 123 and mixing chamber 130 and is provided with an elongated slit 151. This slit has a cross section (i.e. perpendicular to the longitudinal direction of the slit) as shown in Fig. 4, yielding an optimum flow profile. In slit 151 a wedge-shaped valve body 152 is installed, which can be moved by means of a rod 153 in a direction perpendicular to the plane of plate 150. The thickness of valve body 155 corresponds with the smaller dimension of the cross section of slit 151. By moving the valve body, the flow opening between line 123 and mixing chamber 130 can be adjusted with a very high degree of accuracy, the sensitivity depending on the top angle used in the wedge-shaped valve body 152. When valve body 152 is positioned so that an exactly stoichiometric ratio is obtained in the mixing chamber, to be detected by the probe, then the position of the valve body is a measure of the Wobbe index. In so far the operation is identical to what is described with reference to Fig. 4 of Dutch patent application 8802336.

Figure 2 shows another embodiment of a device according to the invention, in which, in the same manner as described above, the probe is used for stoichiometric control of the gas mixture in the combustion chamber. In the embodiment according to Fig. 2 use is made of a pressure control that sets the pressure ratio between natural gas and air in such a way that a stoichiometric mixture is formed in mixing chamber 130. To this end, pressure gauge 140 has been provided, on the side of the natural gas line, with a magnet-coil combination 160, allowing the pressure controller to adjust to a pressure differential between air and natural gas.

Magnet-coil combination 160 exerts a known current-dependent force on the diaphragm. The electrical control of valve 121 keeps the diaphragm in the central position. The variable force exerted on the diaphragm thus results in a variable pressure ratio between gas and air.

Magnet-coil combination 160 is included in a control circuit with the probe, which ensures that the pressure differential is set so that a stoichiometric mixture is formed in the mixing chamber. The current through the coil of the magnet-coil combination then is a measure of the Wobbe index.

A practical embodiment for the supply of air and gas to mixing chamber 130 is shown in Fig. 5. With this configuration optimum mixing can be achieved. The supply system according to Fig. 5 comprises a circular plate 170, which is provided with a number of holes 171 along its circumference. Plate 170, which forms part of mixing chamber 130, covers a cylindrical space 172, formed by a wall 173 and a cylindrical plate 174. Circular plate 174 is provided with a central opening 176, which is connected with line 123 via a line 176. On plate 174 two virtually semi-circular walls, 177 and 178, are formed which, together with wall 173, form two half annular channels which are connected with the central opening 175 via openings 179 and 180, and which on the other side are connected with mixing chamber 130 via the openings 171. From wall 174 further a pipe 181 projects, which extends to plate 170 and which is connected with line 176 via a valve 182. Above the end of pipe 181 an opening 171 is formed in plate 170. Similarly, a second pipe 183 projects from plate 174, which pipe is connected with pressure gauge 140 via a first branch pipe 184, and with the gas line from the coaxial heat exchanger via a second branch pipe 185 and a pipe 186 protruding therefrom. Pipe 186 functions as liquid separator. The gas supplied flows via 185 to pipe 183, while any entrained liquid is discharged via another section of pipe 185.

Figure 7 schematically represents a second embodiment of a pressure gauge 140, the position of diaphragm 143 being detected by a photo-electric system consisting of a light source 190 and two photo-sensitive elements 191 and 192. In addition, in the wall of chamber 141 two lens openings 193 and 194 are provided. In this embodiment diaphragm 143 is in the equilibrium position when both photo-sensitive elements are partially and simultaneously exposed. In all other cases either the one element, 191, or the other, 196, is more exposed, generating a corresponding signal, by which the pressure of the appropriate medium (gas or air) is adjusted.

This renders the output signal of the control independent of any ageing phenomena of the light source or the photo-sensitive element.

Further, in Fig. 7 chambers 143 and 146 are purging chambers, which means that there always is a small flow of gas and air, respectively, through chambers 142 and 143, respectively, so that the most recent gas always is present in chamber 143. This way error measurements are avoided when, in underpressure situations, a part of the gas in chamber 143 would be sucked back into line 124, and thus end up in the mixing chamber.

## Claims

1. Method for determination of the Wobbe index of a gas mixture, the gas mixture being supplied at a first flow rate via at least one flow opening from a supply section to a mixing chamber, an oxidation gas being supplied at a second flow rate via at least one flow opening from a supply section to a mixing chamber, a second gas mixture being formed in the mixing chamber and this second gas mixture being burnt in a combustion chamber, after which the air factor of the resulting combustion gas is measured, characterized in that the pressure of the first gas mixture and that of the oxidation gas just before the mixing chamber are kept in a certain ratio by means of a pressure differential measurement that controls the flow rate of the first gas mixture and/or the oxidation gas, and in that the Wobbe index is determined on the basis of the air factor measured.

2. Method according to claim 1, characterized in that the Wobbe index is determined as a function of the air factor measured absolutely.

3. Method according to claim 1, characterized in that the Wobbe index is determined by controlling the flow rate of the first gas mixture and/or the oxidation gas at a certain air factor and in that the ratio between the quantities used for setting the flow rate of the first gas mixture and/or the oxidation gas is measured for determination of the Wobbe index.

4. Method according to claim 1, characterized in that the pressure ratio is controlled at a constant air factor and in that the Wobbe index is determined on the basis of this pressure ratio.

5. Method according to any one of the preceding claims, characterized in that the first gas mixture and the oxidation gas are brought at the same temperature before they reach the mixing chamber.

6. Method according to claim 5, characterized in that the oxidation gas and the first gas mixture are in heat-exchanging contact while being transported through their respective supply sections.

7. Device for carrying out the method according to any one of claims 1-6.

8. Method substantially as described and represented in the attached drawings.

9. Device substantially as described and represented in the attached drawings.

Fig. 1

Fig. 2

Fig. 5

Fig. 3

Fig. 4

7

Fig. 6

Fig. 7

8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 323 658 (N.V. NEDERLANDSE GASUNIE) * Whole document, in particular column 1, line 2 - column 4, line 2; figures 1,2,4; column 5, line 33 - column 6, line 7; claims 1,3,4,8 * | 1-8 | G 01 N 33/22 G 01 N 31/12 |
| P,X | EP-A-0 326 494 (GAZ DE FRANCE) * Entire document * | 1-4,7,8 ,9 | |
| A | EP-A-0 060 681 (HONEYWELL INC.) * Entire document * | 1-9 | |
| A | EP-A-0 022 493 (RUHRGAS AG) * Entire document * | 1-9 | |
| A | EP-A-0 008 151 (N.V. NEDERLANDSE GASUNIE) * Entire document * | 1-9 | |
| A | EP-A-0 107 341 (HONEYWELL INC.) * Entire document * | 1-9 | TECHNICAL FIELDS SEARCHED (Int. Cl.5) G 01 N 31/00 G 01 N 33/00 |

The present search report has been drawn up for all claims

| Place of search | Date of completion of the search | Examiner |
|---|---|---|
| THE HAGUE | 15-10-1990 | BROCK T.J. |

EPO FORM 1503 03.82 (P0401)